# EUROPEAN PATENT APPLICATION

(11) **EP 3 590 354 A1**
(43) Date of publication of application: **08.01.2020**
(21) Application number: 18181302.3
(22) Date of filing: 02.07.2018
(51) Int. Cl.: A23K 20/105, A23K 50/10, A61K 31/045, A61K 47/10

(54) **FEED ADDITIVE COMPRISING A TRP MODULATOR**

(71) Applicant: PerformaNat GmbH, 14163 Berlin (DE)
(72) Inventor: Rosendahl, Julia, 14163 Berlin (DE); Braun, Hannah-Sophie, 14163 Berlin (DE); Schrapers, Katharina, 14163 Berlin (DE)

(57) **Abstract**

The present invention pertains to a feed additive comprising a TRP modulator and a feed-suitable solvent, wherein the TRP modulator is dissolved in the feed-suitable solvent, and the viscosity of the feed additive is 2,000 cps or less at 20°C.

## Description

The present invention pertains to feed additives comprising a TRP modulator.

In US 2015/0164822, TRP modulators are reported to be used in feed additives. Examples of such TRP modulators include menthol and thymol. Such TRP modulators are generally crystalline and cannot easily be processed or applied to animal feed or animal feed premixes for livestock.

It is an object of the invention to provide for an improved feed additive.

The invention pertains to a feed additive comprising a TRP modulator and a feed-suitable solvent, wherein the TRP modulator is dissolved in the feed-suitable solvent, and the viscosity of the feed additive is 2,000 cps or less at 20°C. By combining the TRP modulator and the feed-suitable solvent the feed additive of the invention can be suitably and easily processed and applied to animal feed or to an animal feed premix. Generally, such feed additives are applied through spraying. The feed additive of the invention can be sprayed more easily and applied at a higher speed compared to feed additives comprising the crystalline TRP modulator per se. The feed-suitable solvent comprised in the feed additive of the invention serves to dissolve the TRP modulator and the solvent may not be easily consumed by the animal, in particular livestock such as ruminants. The TRP modulator, in particular menthol, improves the palatability of the feed additive and hence the uptake of the feed-suitable solvent by the animal. Additionally, the feed-suitable solvent may improve the availability of the TRP modulator, preferably menthol, in the stomach(s) of the animal, which in turn may lead to an improved effect of the TRP modulator, e.g. on anion/cation transfer, in particular Ca²⁺ and/or NH₄⁺, through the stomach wall.

The feed additive of the invention comprises a TRP modulator. The term "TRP modulator" refers to compounds that can influence the activity of the transient receptor potential (TRP) channels. Compound that are capable to increases the activity of the TRP channels are referred to as TRP agonists. TRP antagonists are capable of decreasing the activity of the TRP channels. The TRP modulator can influence one or more specific TRP channels. For instance, the TRP modulator may activate or increase the activity of two or more TRP channels. The TRP modulator may further inhibit or decrease the activity of two or more TRP channels. The TRP modulator may activate one or more TRP channels and may inhibit another TRP channel. Examples of such TRP channels include TRPV (vanilloid receptor) such as TRPV1, TRPV2, TRPV3, TRPV4, TRPV5 and TRPV6; TRPM (melastatin) such as TRPM3, TRPM5, TRPM6, TRPM7 and TRPM8; TRPC (classic) such as TRPC1, TRPC3 and TRPC6; TRPA (ankyrin) such as TRPA1; TRPP (polycystin) such as TRPP1, TRPP2 and TRPP3; and TRPML (mucolipin) such as TRPML1, TRPML2 and TRPML3. Especially interesting for the feed additive of the invention are the TRP channels present in ruminants.

The TRP modulator may be a TRP agonist and/or a TRP antagonist. Examples of TRP modulators include capsaicin, capsazepine, capsiate, capsaicinoids, menthol, eucalyptol, resiniferatoxin, resinifernaoids, piperine, piperidine, camphor, bisandrographolide, 2-aminoethyl-diphenylborinate, icilin, olvanil, verapamil, quinidine, GsMTx4, St. John's wort, hyperforin, ginger, vanillylacetone, evodiamine, gingerol, shogaol, other shogaols, cannabinol, cannabidiol, cannabis, tetrahydrocannabinol, other cannabinoids, polygodal, drimanial, cinnamodial, cinnamosmolide, cinnamolide, afromodial, ancistrodial, merulidial, grifolin, neogrifolin, albaconol, o-prenylphenol, (N-(4-tertiarybutylphenyl)-4-(3-chloropyridin-2-yl)-tetrahydro-pyrazin-1 (2H)-carboxamide, isovelleral, vanillin, vanllotoxin 1, 2 or 3, arvanil, cnidarian envenomations, thapsigargin, yohimbine, AG489 toxin, AG505 toxin, paradol, ginsenoside, mustard oil, allyl isothiocyanate (wasabi), carvacrol, carveol, thymol, borneol, menthone, geraniol, oleocanthal, linalool, isomenthon, menthyl lactate, anethol, p-menthane-3,8-diol, 1-carvone, d-carvone, isopulegol, hydroxyl-citronellal, allicin, cinnamaldehyde (cinnamic aldehyde), methyl salicilate, benzyl isothiocyanate, phenylethyl isothiocyanate, isopropyl isothiocyanate, methyl isothiocyanate, prostaglandin, prostaglandin synthesis inhibitors, acetylsalicylic acid, paracetamol, ibuprofen, warburganal, and combinations of two or more TRP modulators. Preferred TRP modulators are TRP modulators that are crystalline at 20°C. In one embodiment, the TRP modulator is selected from the group consisting of menthol, thymol, cinnamaldehyde, eugenol, anethol, carvacrol, capsaicin, cannabidiol, isomenthon, vanillin, linalool, cannabinol, cannabis, citral and oleocanthal. In another embodiment, the TRP modulator is selected from the group consisting of menthol, thymol, cinnamaldehyde, eugenol, anethol, isomenthon, carvacrol, vanillin and linalool. In a preferred embodiment, the TRP modulator is selected from the group consisting of menthol, thymol, cinnamaldehyde, carvacrol, vanillin and linalool. More preferably, the TRP modulator is selected from the group consisting of menthol, thymol, cinnamaldehyde and carvacrol. In a further preferred embodiment, the TRP modulator is selected from the group consisting of menthol, thymol and cinnamaldehyde. Even more preferably, the TRP modulator is selected from menthol and thymol. Most preferably, the TRP modulator is menthol.

In one embodiment, the feed additive of the invention may comprise the TRP modulator in an amount of at least 1 percent by weight (wt%), preferably at least 5 wt%, more preferably at least 10 wt%, even more preferably at least 15 wt%, and most preferably at least 20 wt%, and preferably at most 90 wt%, more preferably at most 80 wt%, even more preferably at most 70 wt%, even more preferably at most 60 wt%, and most preferably at most 50 wt%, based on the total weight of the feed additive.

In one embodiment, the feed additive of the invention may comprise the TRP modulator in an amount of at least 1 percent by weight (wt%), preferably at least 5 wt%, more preferably at least 10 wt%, even more preferably at least 15 wt%, and most preferably at least 20 wt%, and preferably at most 90 wt%, more preferably at most 80 wt%, even more preferably at most 70 wt%, even more preferably at most 60 wt%, and most preferably at most 50 wt%, based on the total weight of the TRP modulator and solvent.

The feed additive of the invention further comprises a feed-suitable solvent. The feed-suitable solvent is capable of dissolving the TRP modulator, while simultaneously being suitable for use in animal feed. The feed-suitable solvents of the invention may add nutritional value to the animal feed or premix. The solvents, in particular propylene glycol, may also serve as humectant, which improves the uptake of feed by the animal. Moreover, the solvents, in particular propylene glycol, may stabilize the feed additive and/or the feed and may act as a preservative. Examples of suitable solvents include propylene glycol, esters of propylene glycol, polyethylene glycol, molasses, glycerin, esters of fatty acids and combinations of two or more of these solvents. Preferably, the solvent is selected from one or more of the group consisting of propylene glycol, molasses and glycerin. Most preferably, the solvent is propylene glycol.

In one embodiment, the TRP modulator is at least partially dissolved in the solvent of the invention. With "at least partially dissolved" is meant that part of the TRP modulator is dissolved in the solvent, and the other part may be present as solids and thus form a suspension. Preferably, the TRP modulator is completely dissolved in the solvent.

In one embodiment, the feed additive of the invention may comprise the feed-suitable solvent in an amount of at least 10 percent by weight (wt%), preferably at least 20 wt%, more preferably at least 30 wt%, even more preferably at least 40 wt%, and most preferably at least 50 wt%, and preferably at most 99 wt%, more preferably at most 95 wt%, even more preferably at most 90 wt%, even more preferably at most 85 wt%, and most preferably at most 80 wt%, based on the total weight of the feed additive.

In one embodiment, the feed additive of the invention may comprise the feed-suitable solvent in an amount of at least 10 percent by weight (wt%), preferably at least 20 wt%, more preferably at least 30 wt%, even more preferably at least 40 wt%, and most preferably at least 50 wt%, and preferably at most 99 wt%, more preferably at most 95 wt%, even more preferably at most 90 wt%, even more preferably at most 85 wt%, and most preferably at most 80 wt%, based on the total weight of the TRP modulator and solvent.

The feed additive of the invention has a viscosity of 2,000 cps (or cP or mPa.s) or less at 20°C. Preferably, the viscosity of the feed additive is at most 1,000 cps at 20°C, more preferably at most 500 cps, even more preferably at most 200 cps and most preferably at most 100 cps, and preferably at least 1 cps, more preferably at least 5 cps, even more preferably at least 10 cps and most preferably at least 20 cps at 20°C. The viscosity of the feed additive can be determined using any technique commonly used in the art.
Typically, the feed additive should have a viscosity of at most 100 cps for it to be sprayable with conventional sprayers or applicators. The feed additive having a viscosity above 100 cps at 20°C can be heated to obtain a liquid having a viscosity that can be applied with a conventional spray nozzle or spray apparatus used for applying feed additives to animal feed or feed premix.

The feed additive may further comprise other ingredients commonly used in feed additives. Such other ingredients include vitamins and trace minerals.

The feed additive of the invention can be applied to a premix of animal feed or animal feed. Consequently, the invention further pertains to animal feed comprising the feed additive of the invention. More particularly, the invention pertains to animal feed comprising a feed additive comprising a TRP modulator and a feed-suitable solvent, wherein the TRP modulator is dissolved in the feed-suitable solvent, and the viscosity of the feed additive is 2,000 cps or less at 20°C.

In yet another aspect, the amount of feed additive is at least 0.01 wt%, preferably at least 0.1 wt%, more preferably at least 0.5 wt% and most preferably at least 1 wt%, and at most 10 wt%, preferably at most 8 wt%, more preferably at most 5 wt%, and most preferably at most 3 wt%, based on the total weight of the animal feed.

In a first embodiment of the animal feed according to the invention, the feed additive is present in the animal feed in an amount of at least 0.01 kg per ton of total daily intake, preferably at least 0.02 kg, more preferably at least 0.05 kg per ton, and at most 10 kg per ton of total daily intake of feed, preferably at most 5 kg per ton, more preferably at most 2 kg per ton, and most preferably at most 1 kg per ton of total daily intake. In the context of this application, the "total daily intake of feed" is the complete mass of feed an animal takes per day, excluding drinking water and added water to the animal feed.

The invention further pertains to a premix of animal feed comprising a feed additive comprising a TRP modulator and a feed-suitable solvent, wherein the TRP modulator is dissolved in the feed-suitable solvent, and the viscosity of the feed additive is 2,000 cps or less at 20°C.

In yet a further aspect, the amount of feed additive is at least 0.1 wt%, preferably at least 0.5 wt%, more preferably at least 1 wt% and most preferably at least 2 wt%, and at most 15 wt%, preferably at most 12 wt%, more preferably at most 10 wt%, and most preferably at most 5 wt%, based on the total weight of the premix of animal feed.

In yet a further aspect, the amount of TRP modulator is at least 0.1 wt%, preferably at least 0.5 wt%, more preferably at least 1 wt% and most preferably at least 2 wt%, and at most 15 wt%, preferably at most 12 wt%, more preferably at most 10 wt%, and most preferably at most 5 wt%, based on the total weight of the premix of animal feed.

Animal feed generally comprises animal nutrients such as fats and/or proteins and/or carbohydrates that is fed to an animal to provide in its metabolic requirements. Animal feed can be a nutritionally complete feed (i.e. providing all required nutrients to support a normal metabolism of the animal). Similar ingredients are also contained in a premix of animal feed, which however contains only part of the required nutrients, and need to be mixed with other nutrients or fed separately from these other nutrients.

The invention further pertains to a method of feeding an animal by providing feed to the animal comprising a feed additive comprising a TRP modulator and a feed-suitable solvent, wherein the TRP modulator is dissolved in the feed-suitable solvent, and the viscosity of the feed additive is 2,000 cps or less at 20°C.

It was found that the feed additive of the invention, preferably the feed additive comprising menthol, causes an increase in the activity of glutamine synthetase, which in turn leads to an improved nitrogen use efficiency and a better feed conversion. Consequently, the milk of ruminants such as e.g. cows reveal a higher protein level of for example alpha-lactalbumin, beta-lactoglobulin, immunoglobulin, bovine serum albumin, plasmin/plasminogen, lactoferrin and lactoperoxidase. Moreover the milk generally exhibits a higher casein level such as alpha-1-casein, alpha-2-casein, beta-casein and kappa-casein. Furthermore, a higher level of the essential amino acids is generally observed. The milk further contains less urea compared to milk from untreated ruminants (cows). An improved nitrogen use efficiency is further observed in the blood plasma which reveals a lower urea level. Also a lower β-hydroxybutyrate has been observed as well as an increase Ca²⁺ level. In the urine the Ca²⁺ and urea levels are not affected, while the Mg²⁺ and creatinine levels decrease. Additionally, the pH of the urine increases and ammonia excretion is lowered. Therefore, the invention further pertains to the use of the feed additive of the invention, preferably the feed additive comprising menthol, to increase the nitrogen use efficiency of feed, in particular by livestock, preferably by ruminants and most preferably by cows. In a further aspect, the invention pertains to the use of a TRP modulator, preferably menthol, to increase the nitrogen use efficiency of feed, in particular by livestock, preferably by ruminants and most preferably by cows.
The invention further pertains to the use of the feed additive of the invention, preferably the feed additive comprising menthol, to increase the protein and/or casein levels in milk of ruminants, preferably of cows. In a further aspect, the invention pertains to the use of the TRP modulator of the invention, preferably menthol, to increase the protein and/or casein levels in milk of ruminants, preferably of cows. In yet a further aspect, the invention pertains to milk having a protein level exceeding 3.5 g per 100 g milk and/or a casein level exceeding 2.7 g per 100 g milk. Preferably, the milk has a protein level exceeding 3.7 g per 100 g milk and/or a casein level exceeding 3 g per 100 g milk.
The increased nitrogen use efficiency and improved feed uptake by the animal may render cost savings in the feed costs as less feed is necessary.

The increased activity of glutamine synthetase may further lead to a higher availability of glutathione, which is an anti-oxidant, and which protects cells from oxidative stress, e.g. by oxidative radicals. The increased glutamine content may prevent glutamine deficiencies which are observed when giving birth, in metabolic disorders or with injuries. Therefore, the invention further pertains to the use of the feed additive of the invention, preferably the feed additive comprising menthol, to increase the glutamine content in serum, in particular in livestock, preferably in ruminants and most preferably in cows. Moreover, the invention pertains to the use of the TRP modulator of the invention, preferably menthol, to increase the glutamine content in serum, in particular in livestock, preferably in ruminants and most preferably in cows.

In the context of this application, the wording "livestock" refers to animals that are domesticated and raised in an agricultural setting to produce labor and commodities such as meat, eggs, milk, fur, leather and wool. Livestock includes ruminants such as cows, sheep, goats and camels; and monogastrics such as pigs and poultry.

The invention is illustrated with the following examples.

### EXAMPLES

### Example 1

### Experimental Procedures, Animals and Diet

A feeding study was performed with 72 dairy cows (breed: Friesian-Holstein) in the mid-to end lactation separated in two equal groups. The animals fed a total mixed ration (TMR) based on grass maize silage, hay and concentrate feed (Table 1). Cows were milked twice daily; daily milk yield was measured and milk components were measured twice a week. The study aimed to test the effect of a commercial blend of essential oils (BEO) (BTX12, PerformaNat GmbH, Berlin, Germany) offered to dairy cows. A premix of 25g was added to the concentrate feed, which was added to the TMR. In total, animals were fed a daily dose corresponding to 1.2g BEO, with menthol as the major active compound (>80%) in addition to smaller amounts of eugenol and anethol. Groups were fed in a 2x2 cross over design for 20 days with a washout time between test intervals if 40 days.

**Table 1 Diet ingredients and chemical composition of the total mixed ration for dairy cows**

| Feed items (% of dry matter) | |
|---|---|
| Corn silage | 35.2 |
| Grass silage | 24.1 |
| Wheat straw | 2.1 |
| Rapeseed meal | 13.3 |
| Soybean meal | 3.4 |
| Grain of maize | 5.9 |
| Dried sugar beet pulp | 7.0 |
| Rye grains | 8.0 |
| Urea | 0.1 |
| Vitamin premix | 0.2 |
| Feed lime | 0.6 |
| Cattle salt | 0.2 |

| Chemical composition (g/kg dry matter) ¹ | |
|---|---|
| Dry matter | 411 |
| Crude Protein | 153 |
| Crude Fat | 33 |
| Starch | 179 |
| NDF | 389 |
| ADF | 217 |
| ADL | 25 |
| Calcium | 6.1 |
| Magnesium | 2.1 |
| Phosphor | 3.8 |

| | |
|---|---|
| ¹ chemical composition was analysed in three samples of the total mixed ration | |

### Results

### Feed intake and milk parameters

Supplemental BEO did not affect dry matter feed intake (DMI) (Table 2). Milk yield (milk, fat corrected milk (FCM) and energy corrected milk (ECM)) was significantly increased by BEO feeding. Feed efficiency (ECM/DMI and FCM/DMI) significantly increased for cows receiving BEO supplementation, milk yield per DMI tended to increase (*P* = 0.066, Table 2). Fat and protein content of the milk was not influenced by BEO. Fat and protein yield were significantly higher for the BEO group (*P* = 0.007 and *P* = 0.003, respectively). Urea in milk significantly decreased in the BEO group (*P* = 0.006, Table 2).

### Blood parameters

Feeding BEO was associated with a significant rise in plasma calcium levels (control: 2.46 ± 0.015 mmol/l, BEO: 2.53 ± 0.02 mmol/l, *P* < 0.001) (Table 3). No significant changes were observed in serum levels of magnesium, phosphorus, total protein or creatinine. β-hydroxybutyrate significantly decreased in the BEO group (control: 0.78 ± 0.02 mmol/l, BEO: 0.65 ± 0.02 mmol/l, *P* = 0.001), even though cows in mid to late lactation state were not prone to ketosis. Plasma urea levels dropped significantly when BEO was fed (*P* < 0.001, Table 3).

### Urine parameters

In urine, concentrations of calcium and urea were not affected by BEO feeding (**Table 4**). Urinary excretion of magnesium and creatinine tended to decrease in the BEO feeding group (*P* = 0.09 and 0.08, respectively). Urinary pH significantly increased (control: 8.03 ± 0.014 mmol/l, BEO: 8.07 ± 0.014 mmol/l, *P* = 0.033). Changes in titratable acid and base were not significant whereas net acid excretion and acid base ratio tended to increase (*P* = 0.082 and *P* = 0.102, respectively). Ammonia excretion was significantly lower in the BEO group (*P* = 0.013).

**Table 2 Response of feed intake, milk parameters and feed efficiency of dairy cows following dietary supplementation with a blend of essential oils (BEO) for 20 days**

| item | Control | BEO | SEM | *P*-value |
|---|---|---|---|---|
| DMI (kg/d) | 19.7 | 19.5 | 0.28 | 0.558 |

| Milk | | | | |
|---|---|---|---|---|
| Milk (kg/d) | 31.8 | 33.0 | 0.32 | 0.014 |
| ECM (kg/d) | 33.0 | 34.6 | 0.39 | 0.004 |
| FCM (kg/d) | 32.5 | 34.0 | 0.42 | 0.007 |
| Fat (%) | 3.25 | 3.79 | 0.05 | 0.352 |
| Fat (kg/d) | 1.15 | 1.23 | 0.02 | 0.007 |
| Protein (%) | 3.39 | 3.40 | 0.02 | 0.586 |
| Protein (kg/d) | 1.06 | 1.11 | 0.01 | 0.003 |
| Urea (mg/l) | 182 | 166 | 3.88 | 0.006 |

| Feed efficiency | | | | |
|---|---|---|---|---|
| kg milk/kg DMI | 1.62 | 1.70 | 0.032 | 0.066 |
| kg ECM/kg DMI | 1.68 | 1.79 | 0.032 | 0.023 |
| kg FCM/kg DMI | 1.65 | 1.76 | 0.033 | 0.023 |

| | | | | |
|---|---|---|---|---|
| DMI = dry matter intake, ECM = energy corrected milk, FCM = fat corrected milk | | | | |

**Table 3 Response of the plasma parameters of dairy cows following dietary supplementation with a blend of essential oils (BEO) for 20 days**

| Item (mmol/l) | Control | BEO | SEM | *P*-value |
|---|---|---|---|---|
| Calcium | 2.46 | 2.53 | 0.02 | <0.001 |
| Magnesium | 0.98 | 0.99 | 0.01 | 0.241 |
| Phosphorus | 1.90 | 1.91 | 0.03 | 0.994 |
| Urea | 4.28 | 3.92 | 0.07 | <0.001 |
| Total protein | 76.0 | 75.5 | 0.32 | 0.215 |
| BHB | 0.78 | 0.65 | 0.02 | 0.001 |
| Creatinine (*µ*mol/l) | 65.6 | 68.6 | 0.80 | 0.986 |

| | | | | |
|---|---|---|---|---|
| BHB= β-hydroxybutyrate | | | | |

**Table 4 Response of urine parameters of dairy cows following dietary supplementation with a blend of essential oils (BEO) for 20 days**

| Item (mmol/l) | Control | BEO | SEM | *P*-value |
|---|---|---|---|---|
| Calcium | 1.50 | 1.98 | 0.24 | 0.163 |
| Magnesium | 13.6 | 12.6 | 0.40 | 0.090 |
| Urea | 214 | 211 | 5.99 | 0.772 |
| Creatinine | 7.78 | 7.29 | 0.20 | 0.080 |
| pH | 8.03 | 8.07 | 0.01 | 0.033 |
| Titratable base | 185 | 191 | 4.12 | 0.316 |
| Titratable acid | 70.7 | 68.7 | 1.80 | 0.451 |
| NH₃ | 15.6 | 12.5 | 0.88 | 0.013 |
| Base:Acid | 2.77 | 2.94 | 0.07 | 0.102 |
| Net Acid Excretion | 99.1 | 109.6 | 4.19 | 0.082 |

### Example 2

1.09 g menthol is heated until it is melted (>35°C). The fluid menthol is then contacted with 1.09 g propylene glycol and cooled down to room temperature. The resulting liquid has a viscosity of 50.4 cps.

Subsequently, the liquid menthol-propylene glycol blend is sprayed onto a premix of animal feed in a commercial feed mixer to create feed onto which the feed additive of the invention is homogeneously distributed.

Crystalline menthol could not be sprayed on the feed using the commercial feed mixer. Application of crystalline menthol onto animal feed leads to a non-homogenous distribution of menthol over the animal feed.

## Claims

1. Feed additive comprising a TRP modulator and a feed-suitable solvent, wherein the TRP modulator is dissolved in the feed-suitable solvent, and the viscosity of the feed additive is 2,000 cps or less at 20°C.

2. Feed additive according to claim 1 wherein the TRP modulator is a TRP agonist.

3. Feed additive according to claim 2, wherein the TRP agonist is menthol.

4. Feed additive according to any one of the preceding claims, wherein the solvent is propylene glycol.

5. Feed additive according to any one of the preceding claims, wherein the viscosity of the feed additive is 100 cps or less.

6. Animal feed comprising the feed additive of any one of the preceding claims.

7. Method to feed an animal by providing feed to the animal comprising a feed additive comprising a TRP modulator and a feed-suitable solvent, wherein the TRP modulator is dissolved in the feed-suitable solvent, and the viscosity of the feed additive is 2,000 cps or less.

8. Use of the feed additive of any one of claims 1 to 5 to increase the nitrogen use efficiency of feed.

9. Use of the feed additive of any one of claims 1 to 5 to increase the glutamine content in plasma.

10. Use of any one of claims 8 and 9 wherein the feed additive comprises menthol.

11. Use of menthol to increase the nitrogen use efficiency of feed.

12. Use of menthol to increase the glutamine content in plasma.
